# EUROPEAN PATENT APPLICATION

(11) **EP 3 348 200 A1**
(43) Date of publication of application: **18.07.2018**
(21) Application number: 17151212.2
(22) Date of filing: 12.01.2017
(51) Int. Cl.: A61B 6/04, A61G 13/06

(54) **PATENT SUPPORT COUCH ASSEMBLY AND DIAGNOSTIC IMAGING SYSTEM**

(71) Applicant: Koninklijke Philips N.V., 5656 AE Eindhoven (NL)
(72) Inventor: The designation of the inventor has not yet been filed
(74) Representative: de Haan, Poul Erik

(57) **Abstract**

The present invention provides a patient support couch assembly comprising a base member, a support couch for supporting a patient, a first pendulum member and a second pendulum member which are connected pivotably with and between the base member and the support couch, a driving mechanism disposed on the base member, a bracket configured to be driven by the driving mechanism to move horizontally back and forth and a link rod connected pivotably to the first pendulum member at one end and to the bracket at the other end. According to the present invention, the support couch of the patient support couch assembly may be lifted faster, has a greater carrying capacity and be lowered to a sufficiently low height.

## Description

### FIELD OF THE INVENTION

The invention relates to patient support structures. More particularly, the present invention relates to a patient support couch assembly adapted to be used with a diagnostic imaging apparatus such as a computerized tomography (CT) apparatus, a magnetic resonance imaging apparatus or the like. The present invention also relates to a diagnostic imaging system comprising the patient support couch assembly.

### BACKGROUND OF THE INVENTION

In order to obtain diagnostic images of a patient with a diagnostic imaging apparatus, it is necessary that the patient is located exactly at a predetermined position inside an opening of a scan gantry of the diagnostic imaging apparatus. For this reason, the diagnostic imaging apparatus has been provided with a patient support couch assembly which is moveable vertically to be in line with the scan gantry, and horizontally in and out of the scan gantry. Various patient support couch assemblies are known for this purpose. However, in known patient support couch assemblies, a driving mechanism for lifting or lowering a support couch of the patient support couch assembly generally occupies a large space above a base member of the patient support couch assembly. As a result, the known patient support couch assemblies have to use small-size and low power electrical motors such that the support couch of the patient support couch assembly can't be lifted quickly and can't carry great weight. Further, the driving mechanism occupies a large space and prevents the support couch of the patient support couch assembly from being lowered to a sufficiently low height suitable for children or patients in a wheelchair.

Thus, there is a need to make improvements on known patient support couch assemblies.

### SUMMARY OF THE INVENTION

According to one aspect of the present invention, it is to provide a patient support couch assembly comprising:
a base member;
a support couch for supporting a patient;
a first pendulum member and a second pendulum member which are connected pivotably with and between the base member and the support couch;
a driving mechanism disposed on the base member;
a bracket configured to be driven by the driving mechanism to move horizontally back and forth; and
a link rod connected pivotably to the first pendulum member at one end and to the bracket at the other end.

According to other aspect of the present invention, it is to provide a diagnostic imaging system comprising a diagnostic imaging apparatus and the patient support couch assembly above-mentioned.

According to the present invention, the driving mechanism is disposed on the base member and drives the bracket in horizontal orientation to move one end of the link rod to lift or lower the couch. In this way, the driving mechanism will occupy very small space under the couch in vertical orientation such that the couch will have more space to be lowered down. Furthermore, there is almost no limitation in the horizontal space and one end of the link rod may move together with the bracket in horizontal orientation in large range to support the large range of couch height change. In addition, a large size electrical motor, especially a long and thin electrical motor, can be used due to the larger horizontal space compared with the vertical space. Thus, the patient support couch assembly according to the present invention may use large-size electrical motors or hydraulic cylinders having a higher power than those of known patient support couch assemblies. As a result, the support couch of the patient support couch assembly according to the present invention may be lifted faster and have a greater carrying capacity. Further, the driving mechanism and the link rod occupying a smaller space and allow the support couch of the patient support couch assembly according to the present invention to be lowered to a sufficiently low height suitable for children or patients in a wheelchair.

These and other objects, features and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

FIG. 1 is a perspective view showing a patient support couch assembly according to a preferred embodiment of the present invention.
FIG. 2 is a perspective view similar to FIG. 1 with a portion of the patient support couch assembly being cut off to show clearly the structure of the patient support couch assembly.
FIG. 3 is a perspective view showing a driving mechanism of the patient support couch assembly according to the preferred embodiment of the present invention.
FIG. 4 is a perspective view showing a base member of the patient support couch assembly according to the preferred embodiment of the present invention.
FIG. 5 is perspective view showing the patient support couch assembly according to the preferred embodiment of the present invention at the lowest position.
FIG. 6 is perspective view showing the patient support couch assembly according to the preferred embodiment of the present invention at the highest position.
FIG. 7 is a perspective view showing a variation of the driving mechanism of the patient support couch assembly shown in FIG. 3.

### DETAILED DESCRIPTION OF THE EXEMPLARY EMBODIMENTS

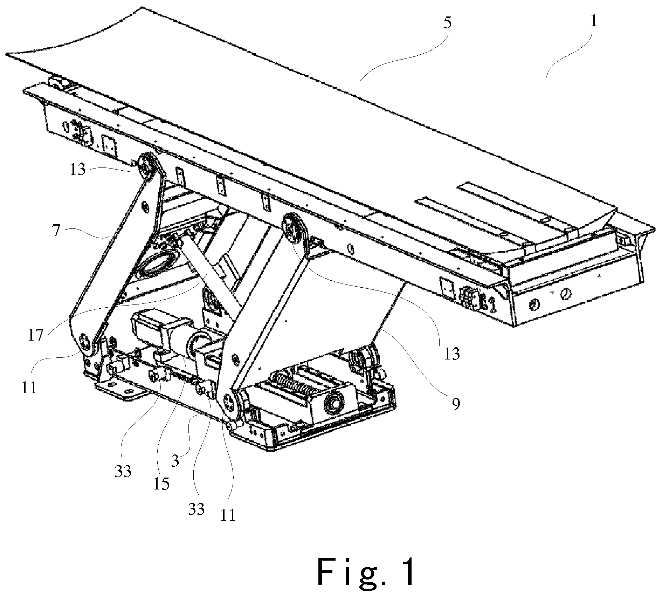

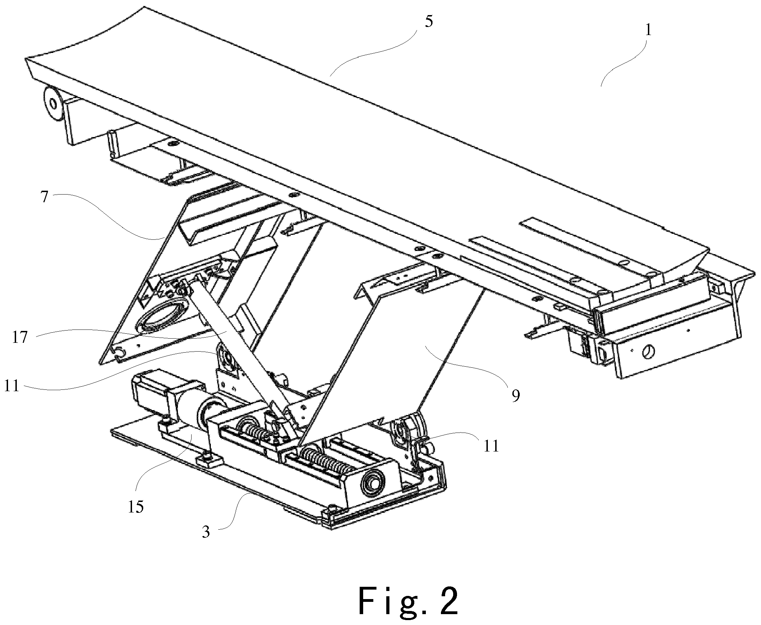

FIG. 1 is a perspective view showing a patient support couch assembly according to a preferred embodiment of the present invention. FIG. 2 is a perspective view similar to FIG. 1 with a portion of the patient support couch assembly being cut off to clearly show the structure of the patient support couch assembly. As shown in Figs 1 and 2, a patient support couch assembly 1 according to the present invention comprises a base member 3, a support couch 5, a first pendulum member 7 and a second pendulum member 9. The base member 3 may be secured to a floor of an examination room or be supported by wheels to move freely on the floor of the examination room. The support couch 5 is configured to support a patient during scanning and imaging. The first pendulum member 7 and the second pendulum member 9 are connected pivotably to the base member 3 at their lower ends by means of first pins 11 and connected pivotably to the support couch 5 at their upper ends by means of second pins 13 so that the support couch 5 may be lifted or lowered with the motion of the first pendulum member 7 and the second pendulum member 9. Although the first pendulum member 7 and the second pendulum member 9 are shown to be in a form of plate in the preferred embodiment, it should be understood that they may be in the form of H-shaped frame or any other suitable form. The patient support couch assembly 1 according to the present invention further comprises a driving mechanism 15 disposed on the base member 3 and a link rod 17 located between the base member 3 and the support couch 5 and configured to connect one of the first pendulum member 7 and the second pendulum member 9 to the driving mechanism 15.

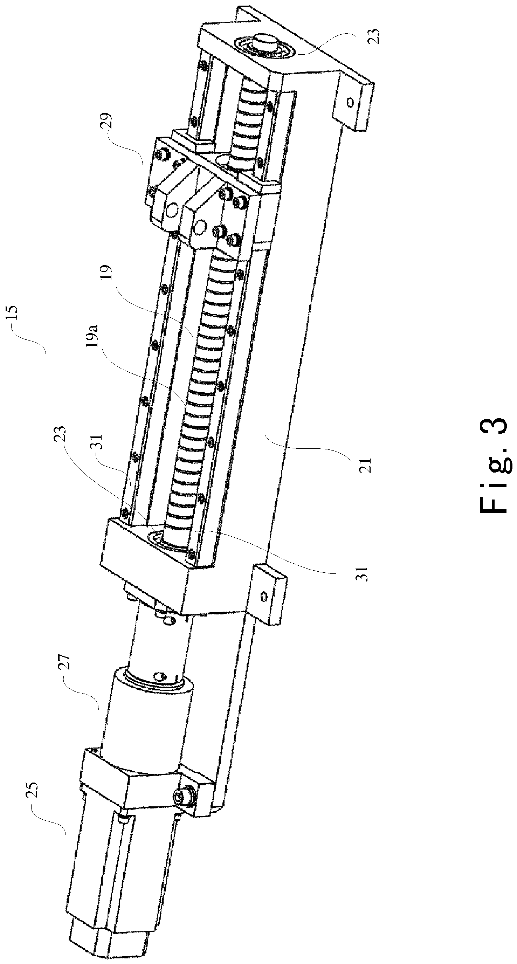

FIG. 3 is a perspective view showing a driving mechanism of the patient support couch assembly according to the preferred embodiment of the present invention. As shown in Fig 3, the driving mechanism 15 comprises a screw 19 supported rotatably on a frame 21 by means of two bearings 23 and an electrical motor 25 for driving the screw 19 to rotate. Preferably, the electrical motor 25 is in line with and coupled to the screw 19 by means of a reducer 27. The patient support couch assembly 1 according to the present invention further comprises a bracket 29 configured to connect link rod 17 to the driving mechanism 15. The bracket 29 has a female thread for engaging with a male thread 19a of the screw 19 so that the bracket 29 may move horizontally back and forth along the screw 19 when the screw 19 is driven by the electrical motor 25 to rotate. Preferably, a guiding rail 31 may be disposed on the frame 21 and the bracket 29 is supported and guided by the guiding rail 31 so that the bracket 29 moves smoothly along the screw 19. In the preferred embodiment shown in Fig 3, there are two guiding rails 31 disposed parallel to each other to guide the bracket 29 to move smoothly along the screw 19.

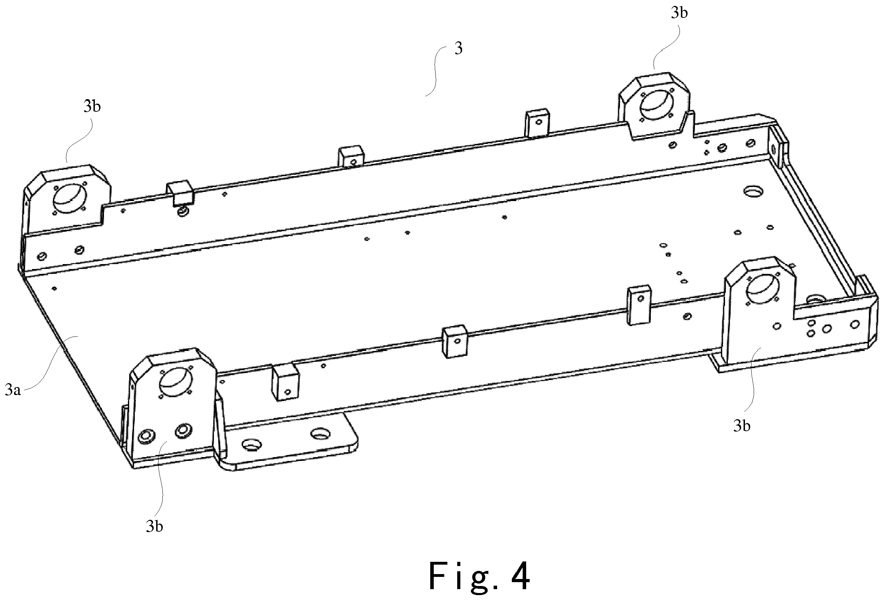

FIG. 4 is a perspective view showing a base member of the patient support couch assembly according to the preferred embodiment of the present invention. The base member 3 comprises a base plate 3a and lugs 3b extending from the base plate 3a to support pivotably the lower ends of the first pendulum member 7 and the second pendulum member 9. The driving mechanism 15 is fixed to the base member 3 by fixing the frame 21 of the driving mechanism 15 to the base plate 3a of the base member 3.

As shown in Figs 1 and 2, the link rod 17 is connected pivotably to the first pendulum member 7 at one end and to the bracket 29 of the driving mechanism 15 at other end. Preferably, the link rod 17 is connected to the first pendulum member 7 at a center of the first pendulum member 7. Also, the screw 19 and the electrical motor 25 are preferably disposed below the link rod 17 such that the structure of the patient support couch assembly according to the present invention is more compact. Although in the preferred embodiment the electrical motor 25 is shown to be disposed below the link rod 17, it should be understood that the electrical motor 25 may be disposed outside of the first pendulum member 7 (i.e. at the left side of the first pendulum member 7) or outside of the second pendulum member 9 (i.e. at the right side of the first pendulum member 9) along a longitudinal axis of the screw 19. More preferably, the electrical motor 25 is disposed outside of the first pendulum member 7 along the longitudinal axis of the screw 19. In this way, the electrical motor 25 will not block downwards motion of the pendulum members 7, 9 so that a larger-size and more powerful electrical motor may be used.

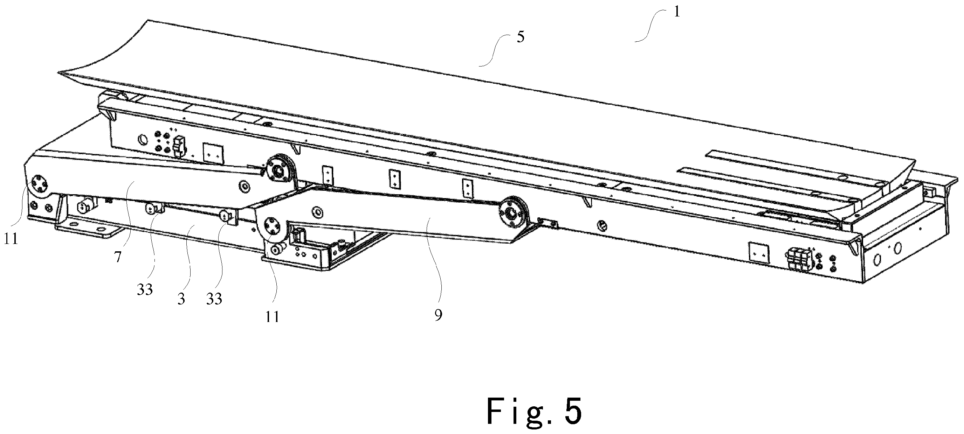

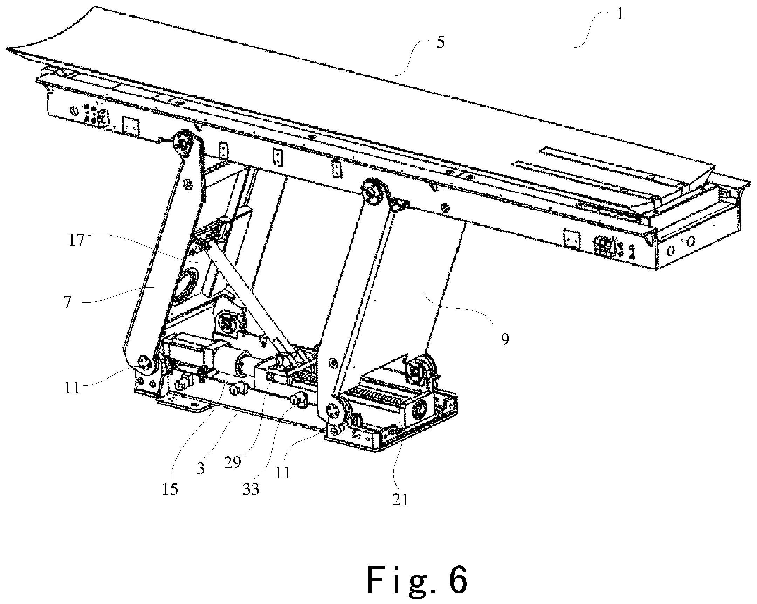

FIG. 5 is perspective view showing the patient support couch assembly according to the preferred embodiment of the present invention at the lowest position. FIG. 6 is a perspective view showing the patient support couch assembly according to the preferred embodiment of the present invention at the highest position. Operation of the patient support couch assembly according to the preferred embodiment of the present invention will be described in detail with reference to Figs 5 and 6. When the electrical motor 25 operates to drive the screw 19 to rotate, the bracket 29 moves along the screw 19 toward one direction by means of thread engagement between the screw 19 and the bracket 29. Supposed the bracket 29 in Figs 5 and 6 moves along the screw 19 toward the right, the link rod 17 moving with the bracket 29 pulls the first pendulum member 7 to pivot clockwise about the first pin 11 toward the base member 3 such that the support couch 5 is lowered gradually until the support couch 5 reaches the lowest position as shown in Fig 5. When the electrical motor 25 reverses and the bracket 29 moves along the screw 19 toward the left, the link rod 17 moving with the bracket 29 pushes the first pendulum member 7 to pivot anticlockwise about the first pin 11 away from the base member 3 so that the support couch 5 is lifted gradually until the support couch 5 reaches the highest position as shown in Fig 6. Of course, the support couch 5 may be stopped at any required position by switching off the electrical motor 25. Preferably, the male thread 19a of the screw 19 takes a form of trapezoidal thread to achieve a self-lock between the screw 19 and the bracket 29, thereby preventing the support couch 5 from falling down when the electrical motor 25 is not in operation. Preferably, stops 33 are disposed on the base member 3 to limit the lowest position of the support couch 5, thereby preventing the support couch 5 from squeezing the link rod 17 and/or the driving mechanism 15.

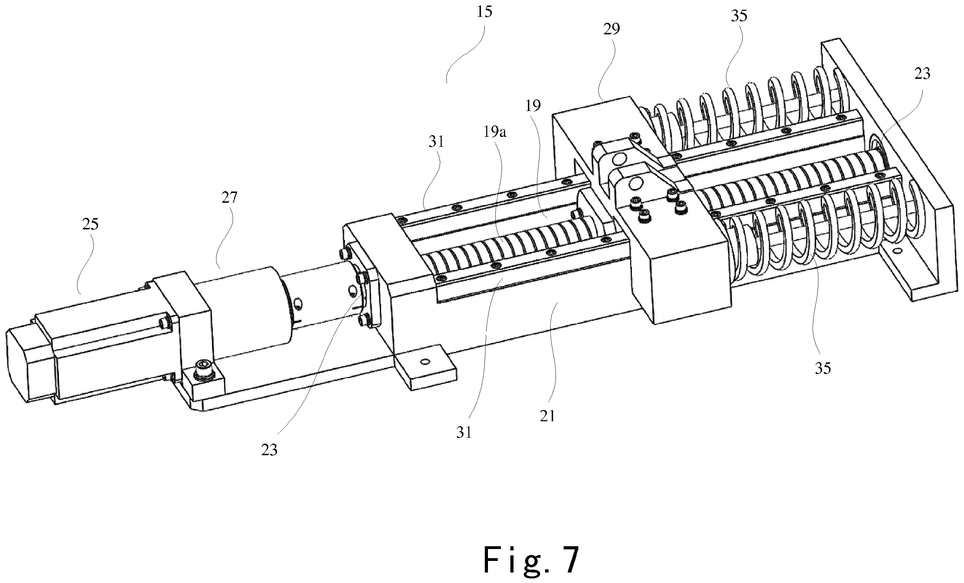

FIG. 7 is a perspective view showing a variation of the driving mechanism of the patient support couch assembly shown in FIG. 3. The variation of the driving mechanism as shown in Fig 7 differs from the driving mechanism as shown in Fig 3 only by further comprising a spring 35 disposed between the bracket 29 and one end of the frame 21. The spring 35 may be a compression spring or an extension spring. However, the spring 35 always tends to apply a force to the bracket 29 that facilitates the lifting movement of the support couch 5, thereby reducing a power required for the electrical motor 25.

Although the driving mechanism in the preferred embodiment comprises the screw and the electrical motor for driving the screw, it should be understood that the driving mechanism may comprise a hydraulic cylinder for actuating the bracket to move back and forth along the guiding rails. Further, although the first pendulum member and the second pendulum member in the preferred embodiment are arranged in a parallelogram structure, it should be understood that the first pendulum member and the second pendulum member may be arranged in a scissor structure as well known in the art. Further, it is possible for a person skilled in the art to envisage that the base member and the frame may be formed integrally.

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims.

## Claims

1. A patient support couch assembly (1) comprising:
a base member (3);
a support couch (5) for supporting a patient;
a first pendulum member (7) and a second pendulum member (9) which are connected pivotably with and between the base member (3) and the support couch (5);
a driving mechanism (15) disposed on the base member (3);
a bracket (29) configured to be driven by the driving mechanism (15) to move horizontally back and forth; and
a link rod (17) connected pivotably to the first pendulum member (7) at one end and to the bracket (29) at the other end.

2. The patient support couch assembly (1) according to claim 1, wherein the driving mechanism (15) comprises a screw (19) supported rotatably on a frame (21) and an electrical motor (25) for driving the screw (19) to rotate, the bracket (29) has a female thread for engaging with a male thread (19a) of the screw (19) so that the bracket (29) moves back and forth along the screw (19) when the screw (19) rotates.

3. The patient support couch assembly (1) according to claim 2, wherein the male thread (19a) of the screw (19) takes a form of trapezoidal thread to achieve a self-lock between the screw (19) and the bracket (29).

4. The patient support couch assembly (1) according to claim 2, wherein a guiding rail (31) is disposed on the frame (21) to guide the bracket (29) to move along the screw (19).

5. The patient support couch assembly (1) according to claim 2, wherein the electrical motor (25) is in line with and coupled to the screw (19) by means of a reducer (27)

6. The patient support couch assembly (1) according to claim 2, wherein the screw (19) and the electrical motor (25) are disposed below the link rod (17).

7. The patient support couch assembly (1) according to claim 2, wherein the electrical motor (25) is disposed outside of the first pendulum member (7) along the longitudinal axis of the screw (19).

8. The patient support couch assembly (1) according to claim 1, wherein the driving mechanism (15) comprises a hydraulic cylinder for actuating the bracket (29) to move back and forth along a guiding rail.

9. The patient support couch assembly (1) according to claim 1, wherein a stop (33) is disposed on the base member (3) to limit the lowest position of the support couch (5).

10. The patient support couch assembly (1) according to claim 1, wherein the patient support couch assembly (1) further comprises a spring (35), the spring (35) tends to apply to the bracket (29) a force that facilitates a lifting movement of the support couch (5).

11. The patient support couch assembly (1) according to claim 1, wherein the link rod (17) is connected to the first pendulum member (7) at a center of the first pendulum member (7).

12. The patient support couch assembly (1) according to claim 1, wherein the first pendulum member (7) and the second pendulum member (9) are connected between the base member (3) and the support couch (5) in a parallelogram structure or in a scissor structure.

13. A diagnostic imaging system comprising a diagnostic imaging apparatus and a patient support couch assembly (1) according to any one of claims 1-12.
